# EUROPEAN PATENT APPLICATION

(11) **EP 3 777 702 A1**
(43) Date of publication of application: **17.02.2021**
(21) Application number: 19774921.1
(22) Date of filing: 11.01.2019
(51) Int. Cl.: A61B 17/00

(54) **OCCLUSION DEVICE AND PREPARATION METHOD THEREFOR**

(30) Priority: 28.03.2018 CN 201810265864
(71) Applicant: Shanghai MicroPort Medical (Group) Co., Ltd., Shanghai 201203 (CN)
(72) Inventor: KANG, Yahong, Shanghai 201203 (CN); ZHANG, Guoyi, Shanghai 201203 (CN); GAO, Chenguang, Shanghai 201203 (CN); LU, Wei, Shanghai 201203 (CN); JIANG, Hongyan, Shanghai 201203 (CN)
(74) Representative: Dauncey, Mark Peter
(86) International application number: PCT/CN2019/071340
(87) International publication number: WO 2019/184555

(57) **Abstract**

The present invention provides an occlusion device and a preparation method therefor. The occlusion device includes a framework and blood-blocking mem-branes (3, 4). The framework includes a waist (1) and barbs (2, 5) connected to at least one end of the waist (1), and the waist (1) is coated with a solid xerogel. The blood-blocking membranes (3, 4) are secured at least one end of the waist (1) and/or at inner sides of the barbs (2, 5). This occlusion device employs a structure allowing it to have a smaller size and thus be able to be loaded in a thinner delivery sheath having a smaller diameter. Additionally, the amount of material used is reduced, lowering the incidence of post-implantation complications and resulting in cost savings. Taking advantage of the behavior of the solid xerogel that it swells in the body when coming into contact with blood and absorbs water therein, defects with irregular shapes, such as those with relatively narrow edge rims and complicated shapes, can be filled up, thus extending the scope of application of interventional treatment to cardiac septal defects.

## Description

### TECHNICAL FIELD

The present invention relates to medical occlusion device and preparation method therefor and, in particular, to a self-expanding occlusion device for cardiac septal defect and a preparation method therefor.

### BACKGROUND

With the continuing advancement of implantable medical instruments and interventional cardiology, transcatheter percutaneous implantation of occluders has been increasingly important and accepted in the treatment of atrial septal defect (ASD), patent foramen ovale (PFO), ventricular septal defect (VSD), patent ductus arteriosus (PDA) and other cardiac diseases.

Scientifically and clinically, there have been accumulated a wealth of knowledge on such occluders, in particular nickel-titanium alloy occluders that have been put in clinical practice for more than a decade. A cardiac septal defect occluder made of a nickel-titanium alloy has excellent closure performance with minor leakage because its good shape memory properties allow it, when implanted into the body of a patient, to immediately return to the desired shape that snugly fits the defect. However, such occluders are not degradable and will permanently remain in patient's bodies, leaving potential risks. Although there are also occluders made essentially of biodegradable materials, most of them are currently in a research phase. In addition, due to inherent characteristics and features of the biodegradable materials, such an occluder tends to be insufficient in resilience between its two occlusion members and thus not able to ensure their close fits to both sides of the defect, possibly leading to undesirable closure results. For this reason, it is necessary for such an occluder to be designed with some locking structures for maintaining the distance between the disk-like surfaces at a desired value for stabilization. However, existing such locking structures are problematic and deficient and their fabrication is complicated and costly.

Although current implantable occluders are diversified and vary in material, ranging from non-degradable alloys to degradable polymers, and in geometry, they commonly feature a double-disc design including two discs and a thinner waist connecting them. Blood-blocking membranes are sutured onto surfaces of the discs so that when the device is deployed with the discs being sandwiched by and close to the defect, the membranes can block blood from flowing through the defect. However, this design is still associated with a number of deficiencies. For example, each disc spans a relatively large area when it fully expands, requiring, as a basic requisite for the interventional treatment using the occluder, the defect to be placed with its edge spaced from each of the coronary sinus, superior vena cava, inferior vena cava, pulmonary vein and atrioventricular valves by a certain distance that is sufficient to accommodate the expansion. This precludes some patients from such interventional treatment. Besides, such an occluder is bulky and uses a large amount of material. Therefore, whatever material (such as a nickel-titanium alloy or a degradable material) is used, it tends to account for significant later complications and cause great blood vessel damage due to a correspondingly bulky catheter required for delivery of the occlude. Furthermore, different patients vary in terms of defect anatomy, making it impossible for the same occluder shape to address all the requirements for complete closure without significant residual leakage.

### SUMMARY OF THE INVENTION

In view of the above-described problems, there is a need for a occlusion device for closing a defect in the cardiac septum and a preparation method of such an occlusion device, which features good structural stability with less springback or displacement, a compact overall size allowing it to be widely used in interventional treatment of various cardiac septal defects, a small footprint, low material consumption, excellent biocompatibility, fast defect closure and the ability to fill up various anatomically irregular defects.

To this end, the present invention provides an occlusion device, comprising a framework and blood-blocking membranes, the framework comprising a waist and barbs connected to at least one end of the waist, the waist being coated with a solid xerogel, the blood-blocking membranes being secured at least one end of the waist and/or at inner sides of the barbs.

Further, the solid xerogel is made of a degradable material.

Further, the degradable material of the solid xerogel is selected from block or cross-linked copolymers, each consisting of at least one aliphatic polymer and at least one water-soluble polymer or of at least one amphiphilic polymer and at least one aliphatic or water-soluble polymer.

Further, the aliphatic polymer is selected from polylactic acid or a copolymer thereof, or from polycaprolactone or a copolymer thereof, and wherein the water-soluble polymer is selected from polyacrylic acid or polyethylene glycol, and wherein the amphiphilic polymer is selected from poly (N-isopropylacrylamide) or a copolymer thereof.

Further, the degradable material of the solid xerogel has a weight average molecular weight in a range of 10⁴-10⁶ Da.

Further, the framework and/or the blood-blocking membranes are/is each made of a degradable material.

Further, the degradable material of the framework is selected from a degradable magnesium alloy, a degradable zinc alloy or one or a copolymer and/or blend of more of polylactic acid, polyglycolide, polycaprolactone, polydioxanone and polyamino acid, and wherein the degradable material of the blood-blocking membranes is selected from one or a copolymer and/or blend of more of polylactic acid, polyglycolide, polycaprolactone, polydioxanone and polyamino acid.

Further, the waist is a meshed tubular structure or a hollow tubular structure.

Further, the waist has an axial length in a range of 3.5-5.5 mm and an inner diameter in a range of 2-60 mm.

Further, each of the barbs extends along a radial direction of the framework from a junction with the waist away from the waist and comprises at least one inwardly bent section pointing toward the waist.

Further, each of the barbs comprises a plurality of inwardly bent sections including at least one semicircular curved bent section and/or at least one hook-shaped bent section.

Further, the barbs are uniformly or non-uniformly arranged across a circumference of the waist or across one half or three quarters of the waist.

Further, the barbs are arranged on both ends of the waist, and wherein magnetic poles that attract each other are arranged on the barbs which are at symmetrical locations of proximal and distal ends of the waist respectively.

The present invention provides a preparation method for the above occlusion device, comprising steps of:
forming the waist of the framework by braiding filaments or laser-cutting a tube;
forming the barbs at at least one end of the waist by heat-setting;
securing the blood-blocking membranes at at least one end of the waist and/or at inner sides of the barbs; and
coating the solid xerogel onto the waist.

Further, the waist is formed by braiding filaments, and wherein the barbs are formed by heat-setting portions of the filaments reserved at the end(s) of the waist.

Further, the heat-setting comprises bending the filaments toward the waist by an over-pressure and thus imparting inward pre-stress to the barbs.

Further, coating the solid xerogel onto the waist comprises: placing the waist in a hydrogel and forming the solid xerogel over the waist by freeze-drying the hydrogel; or spraying the hydrogel onto the waist and obtaining the solid xerogel by drying the hydrogel through solvent evaporation; or spraying the hydrogel onto the filaments or the tube from which the waist is formed and obtaining the solid xerogel by drying the hydrogel through solvent evaporation.

Further, the blood-blocking membrane are secured at at least one end of the waist and/or at inner sides of the barbs by suturing, hot-melting or gluing.

The present invention has the following benefits over the prior art: there provides an occlusion device and a preparation method therefor, the occlusion device employing a structure allowing it to have a smaller size and be loaded in a thinner delivery sheath having a smaller diameter. As a result, damage to the wall of a blood vessel in which the sheath is inserted can be reduced, and interventional closure treatment of younger patients is made possible. Additionally, the amount of material used is reduced, lowering the incidence of post-implantation complication and resulting in cost savings. Taking advantage of the behavior of the solid xerogel that it swells in the body when coming into contact with blood, defects with irregular shapes, such as those with relatively narrow edge rims and complicated shapes, can be filled up, thus extending the scope of application of interventional treatment to cardiac septal defects.

### BRIEF DESCRIPTION OF THE DRAWINGS

Fig. 1 is a schematic view illustrating the overall structure of an occlusion device in a released configuration according to an embodiment of the present invention.
Fig. 2 is a front view of an occlusion device in a released configuration according to an embodiment of the present invention.
Fig. 3 is a top view of an occlusion device in a released configuration according to an embodiment of the present invention.
Fig. 4 is a schematic illustration of an occlusion device that has been released from a catheter according to an embodiment of the present invention.
Fig. 5 is a schematic illustration of an occlusion device that is closing a cardiac septal defect according to an embodiment of the present invention.

In these figures, 1 denotes a waist; 2, proximal barbs; 3, a proximal blood-blocking membrane; 4, a distal blood-blocking membrane; 5, distal barbs; 8, an occlusion device; 9, tissue wall around a defect; 10, a delivery catheter; and 11, a defect.

### DETAILED DESCRIPTION

The present invention will be described in greater detail below with reference to specific embodiments and the accompanying drawings.

In order to more clearly describe structural features of the present invention, the directional and orientational terms "proximal" and "distal" will be used herein. For example, a "proximal end" refers to an end nearer to an operator during a surgical procedure, while a "distal end" refers to an end farther away from the operator. As used herein, the term "or" is generally employed in its sense including "and/or", unless the content clearly dictates otherwise.

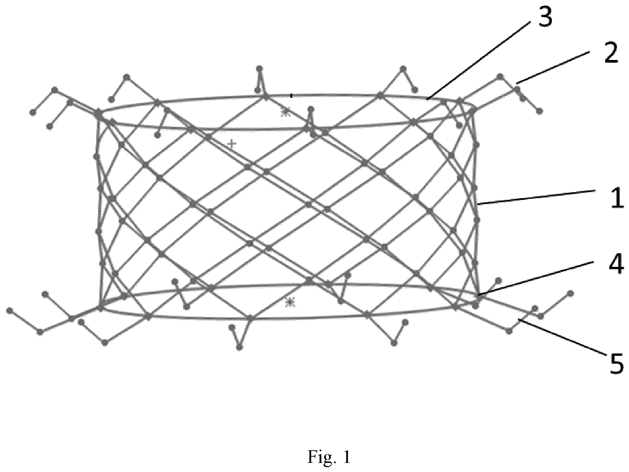

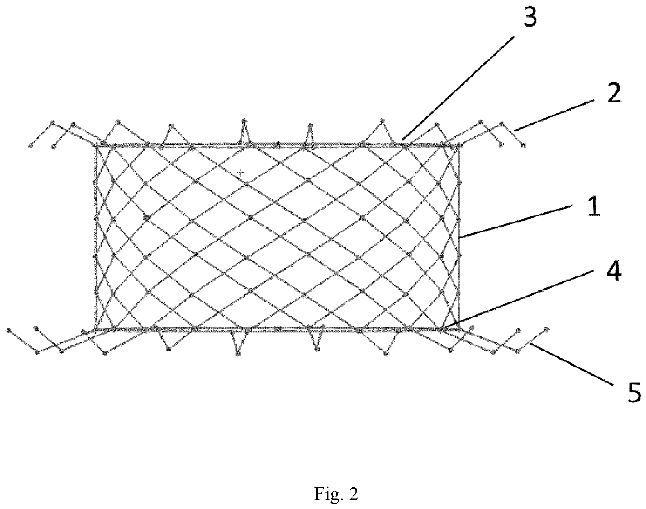

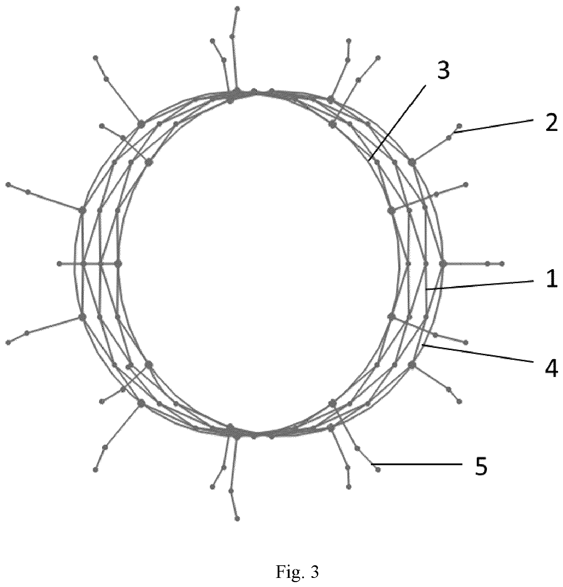

Fig. 1 is a schematic view illustrating the overall structure of an occlusion device 8 in a released configuration according to an embodiment of the present invention. Fig. 2 is a front view of the occlusion device 8 in the released configuration according to an embodiment of the present application, and Fig. 3 is a top view of the occlusion device 8 in the released configuration according to an embodiment of the present application.

The occlusion device 8 includes a framework, blood-blocking membranes and a solid xerogel serving as a defect filling medium. Such occlusion device 8 features good structural stability with less reversion or displacement, a compact overall size, low material consumption, excellent biocompatibility, fast closure of an anatomically irregular defect as a result of quick swelling of the solid xerogel upon contact with blood and easy operability. Additionally, it solves problems in conventional occlusion device, such as a bulky volume that creates difficulties in delivery in a sheath through a thin blood vessel, as well as unsuitability for patients with defects having narrow edge rims or complicated shapes.

Referring to Figs. 1 through 3, the occlusion device 8 includes the framework, the solid xerogel and the blood-blocking membranes 3, 4. The framework includes a waist 1 and proximal barbs 2 or distal barbs 5 that are connected to at least one end of the waist 1 and configured to anchor the occlusion device 8. The blood-blocking membranes are secured to the framework and, in one embodiment, include a proximal blood-blocking membrane 3 and a distal blood-blocking membrane 4, which are fixed to proximal and distal ends of the waist 1, or to the inner sides of the proximal and distal barbs 2, 5 (i.e., on the side of the barbs closer to a center axis of the waist 1), respectively. In another embodiment, only the proximal blood-blocking membrane 3 or distal blood-blocking membrane 4 is included. In the illustrated embodiment, the solid xerogel is coated on the waist 1 and, after the occlusion device 8 is successfully implanted, quickly swells and fills up and close a defect 11 when it contacts with blood and absorbs water therein. Coating the solid xerogel on the waist does not preclude the possibility of coating it on other components such as the barbs or blood-blocking membranes. The framework, solid xerogel and blood-blocking membranes are preferably made of degradable materials.

Preferably, the framework is formed of a degradable material capable of imparting to the framework desired mechanical properties and a controllable degradation period, satisfying predetermined strength requirements and maintaining the shape of the framework in a short term without reversion. Preferably, the degradable material is selected from one or a copolymer and/or blend of more of polylactic acid (PLA), polyglycolide (PGA), polycaprolactone (PCL), polydioxanone (PDO) and polyamino acid (PAA). For example, it may be preferably a PLA-PCL copolymer, a PLA-PGA copolymer or a PLA-PCL-PGA copolymer. It may also be selected from a degradable metal and alloy, such as a degradable magnesium alloy and a degradable zinc alloy. Preferably, the degradable material forming the framework has a weight average molecular weight in the range of 10⁵-10⁶ Da and has a distribution width of 1-3.

The solid xerogel is a dehydrated form of a hydrogel. When brought into contact with water, the solid xerogel can quickly absorb the water and swell back into the hydrogel with a significantly increased volume. The solid xerogel has good biocompatibility and tissue adhesion and is preferably made of a degradable material selected from block or cross-linked copolymers each consisting of at least one aliphatic polymer and at least one water-soluble polymer or of at least one amphiphilic polymer and at least one aliphatic or water-soluble polymer. Preferably, the aliphatic polymer is selected from PLA or a copolymer thereof, or from PCL or a copolymer thereof. The water-soluble polymer is selected from polyacrylic acid (PAA) or polyethylene glycol (PEG). The amphiphilic polymer is selected from poly (N-isopropylacrylamide) (PNIPAAm) or a copolymer thereof. The block or cross-linked copolymers may include PEG-PLGA-PEG, PLGA-PEG-PLGA, PCLA-PEG-PCLA, Dex-PCL-HEMA or the like. Preferably, the degradable material from which the solid xerogel is formed has a weight average molecular weight in the range of 10⁴-10⁶ Da. In particular, a degradation period and mechanical properties of the degradable solid xerogel may be adjusted by changing its constituent polymers' molecular weights, ratio(s) or the like. It may be controlled to start degrading slowly about 6 months later after the completion of endothelialization.

In particular, the solid xerogel may be obtained by: placing the framework (including the waist 1) in the hydrogel and then freeze-drying the hydrogel into the xerogel; or spraying the hydrogel onto the waist 1 and then drying the hydrogel into the xerogel through solvent evaporation; or spraying the hydrogel onto a wire or tube for forming the framework (including the waist 1) and then shaping the wire or tube into the framework.

As noted above, the framework includes the waist 1 and the proximal barbs 2 or distal barbs 5 that are connected to at least one end of the waist 1 (e.g., the barbs are provided at only one end of the occlusion device 8, when the occlusion device 8 is used for PDA closure) and configured to anchor the occlusion device 8. The framework may be a meshed tubular structure, for example, braided from monofilaments made of the aforementioned degradable material. A proper length of the tubular structure may be selected as the waist 1 according to a target implantation site, with monofilaments at one or both ends of the waist 1 being subsequently heat-set into the barbs. Depending on the defect to be closed, the barbs may be formed at either one or both ends of the waist 1. For example, when the occlusion device 8 is used for patent ductus arteriosus (PDA) closure, the barbs 5 are usually provided only at the distal end of the waist 1. In another embodiment, the meshed tubular structure (similar to a cut stent) may also be fabricated by laser-cutting a tube (or a bar) made of the degradable material into the waist 1 and then forming the barbs at one or both ends of the waist 1 by performing processes such as heat treatment, finishing, etc. Alternatively, the waist 1 of the framework may also be a hollow tubular structure without through openings or holes on the tubular wall, which is fabricated from a tube made of the degradable material.

The waist 1 functions essentially to connect the proximal barbs 2 and/or distal barbs 5 so that the occlusion device 8 is fitted against the defect and maintained in position, and to provide a support for the blood-blocking membranes 3, 4 and the solid xerogel. The waist 1 may be suitably flexible so that, even when the defect is complicated in shape, it can still conform to a surrounding wall thereof by properly deforming.

Preferably, the waist 1 has a length of about 3.5-5.5 mm, preferably 4 mm, which is equivalent to a wall thickness of the defect (e.g., cardiac septal defect). In addition, an inner diameter of the waist 1 may be designed to range from 2 mm to 60 mm, depending essentially on a size of the cardiac septal defect. However, it should be significantly greater than a diameter of the cardiac septal defect so that, when in an expanded configuration, the waist 1 can be closely pressed against the defect's inner surface. The occlusion device may be produced in various sizes to address the needs of different patients.

Each of the proximal and distal barbs 2, 5 extends along a radial direction of the framework from the junction with the waist away therefrom and has at least one bent section pointing back toward the waist. It may be designed to various shapes. For example, it may have only one bend so that it can directly press against and anchor on tissue around the defect. In this way, the overall structure is compact and has a small footprint. As another example, each of the proximal and distal barbs 2, 5 may have multiple bends, which for example include at least one semicircular curved bend and at least one hook-shaped bend so as to press against and pierce into the tissue, thus effectively preventing displacement of the occlusion device 8. The proximal and distal barbs 2, 5 may be formed by heat-setting.

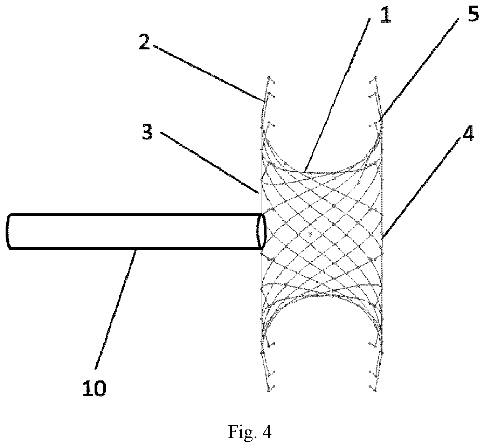

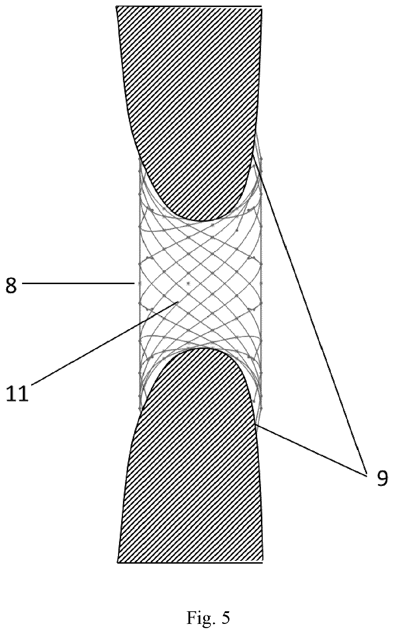

Referring to Figs. 4 and 5, when the occlusion device 8 is delivered through a delivery catheter 10 to the target site and expands thereat, the proximal and distal barbs 2, 5 will hook on the tissue, thus securing the occlusion device 8 in place. In order to achieve better anchoring of the barbs 2, 5 without any potential displacement, one of the following approaches is preferred: a) adding magnetic poles that attract each other to barbs symmetrically located at the proximal and distal ends of the waist respectively, thus allowing an attraction between the barbs, which reinforces the fixation of the occlusion device 8; and b) during the formation of the proximal and distal barbs 2, 5 by heat-setting, over-pressing them with an inward pre-stress toward the center axis of the waist, which can significantly strengthen the clamping and fixation of the occlusion device 8.

The number and sizes of the proximal or distal barbs 2, 5 may be designed according to an edge rim condition of the defect. Depending on how distant the edge rim of the defect is from the coronary sinus, superior vena cava, inferior vena cava, pulmonary vein and atrioventricular valves, the barbs may be distributed in different fashions, for example, uniformly or non-uniformly across a circumference of the waist 1 or across one half or three quarters thereof. Preferably, the barbs are uniformly distributed across the aforementioned length.

In particular, when the edge rim of the defect is relatively close to the coronary sinus, superior vena cava, inferior vena cava, pulmonary vein or atrioventricular valves, the barbs are arranged only at the proximal or distal end that is farther away from the veins or valves, in order to prevent them from coming into contact with the veins or valves and possibly causing an acute reaction.

The blood-blocking membranes are secured at at least one end of the waist or at inner sides of the barbs. They may be secured at either one or both ends of the waist or at inner sides of the proximal or distal barbs. Preferably, the blood-blocking membranes are secured at at least one end of the waist or inner sides of the barbs by suturing, hot-melting or gluing. They function mainly to block the blood from flowing therethrough and may include a proximal blood-blocking membrane 3 secured at a proximal end of the waist 1 or at inner sides of the proximal barbs 2 and a distal blood-blocking membrane 4 secured at a distal end of the waist 1 or at inner sides of the distal barbs 5. Preferably, the blood-blocking membranes are made of a degradable material selected from one or a copolymer and/or blend of more of polylactic acid (PLA), polyglycolide (PGA), polycaprolactone (PCL), polydioxanone (PDO) and polyamino acid (PAA). For example, the degradable material of the blood-blocking membranes may be made from a degradable polymer such as a PLA-PCL copolymer, a PLA-PGA copolymer, a PLA-PCL-PGA copolymer or the like. Preferably, the degradable material of the blood-blocking membranes has a weight average molecular weight in the range of 10⁵-10⁶ Da and a distribution width of 1-3.

In order to accurately trace the occlusion device 8 during or after the implantation, a traceable marker may be added or secured to the occlusion device 8 in one of a number of ways. In one embodiment of the present invention, where the waist of the framework is a meshed tubular structure with both itself and the barbs being braided from monofilaments, a number of traceable filaments may be mixed with the monofilaments to fabricate the meshed tubular framework. Alternatively, a traceable material may be sutured onto a proper location of the occlusion device 8. Yet alternatively, the traceable material may be provided in one or more depressions engraved in the framework. Still yet alternatively, the traceable material may be blended in a suitable amount and co-extruded with the degradable material to form the occlusion device 8. All of these approaches can accomplish the securing of the traceable marker so that it can be determined that whether the occlusion device 8 is precisely deployed at the cardiac defect or whether it has experienced any displacement thereafter.

The traceable material of the traceable marker may be selected from: non-metallic substances, such as diatrizoic acid, sodium iodide, iohexol, iodixanol, Ioversol, barium sulfate, meglumine diatrizoate, bismuth subcarbonate, titanium ox-ide, zirconium oxide, etc.; and metallic substances, such as gold, rhenium, iridium, molybdenum, tungsten, platinum, rhodium, etc. The traceable material may be either a biodegradable substance, such as iron, gold, rhenium, iridium, molybdenum, etc., or a non-biodegradable substance such as meglumine diatrizoate, iopromide, iohexol, etc.

Each component of the occlusion device 8 is preferably made of a degradable material in order to provide good biocompatibility. In addition, some of the components in the occlusion device 8 may be each selected from a number of suitable implementations, as practically required, and combined freely to form the occlusion device 8.

### EBODIMENT 1

An example of the occlusion device 8 will be explained below, where it is a meshed tubular structure with multiple barbs, formed by braiding and used to close a central ASD. Reference is specifically made to Figs. 1 to 5.

In this embodiment, the occlusion device 8 includes a framework, blood-blocking membranes and a traceable marker. The framework includes a waist 1 and proximal barbs 2 and distal barbs 5 connected to the respective ends of the waist 1 and configured to secure the occlusion device 8.

In this embodiment, the waist 1 is a meshed tubular structure braided from monofilaments with end portions for forming the barbs 2, 5 being reserved at the respective ends of the waist 1. The traceable marker is formed of traceable filaments made of a degradable material, which are braided with the degradable filaments symmetrically with respect to an axis of the waist 1. The traceable filaments can be used to determine whether the occlusion device 8 is precisely positioned at the cardiac defect and whether it experiences any displacement thereafter.

The monofilaments from which the waist 1 and barbs 2, 5 are formed may be made of a degradable polymer treated by a set of processes including stretching, braiding and heat-setting. In this embodiment, the meshed tubular structure is braided from poly-L-lactic acid (PLLA) monofilaments, and a portion of the meshed tubular structure having a length of about 4 mm serves as the waist 1 (since a central ASD usually has a wall thickness of about 4 mm). The barbs are formed by performing processes including heat-setting and finishing on portions of the monofilaments reserved at both ends of the waist 1. Each barb may be formed of two or more monofilaments and referred to as a proximal barb 2 or distal barb 5 depending on where it is located relative to the waist 1. The barbs 2, 5 extends from their junctions with the waist 1 radially with respect to the occlusion device 8 away from the waist 1 and each possess inward pre-stress obtained through bending them inwardly (i.e., toward the waist) with an over-pressure. During closure, the barbs 2, 5 in an expanded configuration clamp tissue wall around the defect from both sides thereof and hook thereon, thus ensuring that the occlusion device 8 is firmly secured without any possible displacement.

After the fabrication of the framework is completed, a solid xerogel is coated on the waist 1. This may include: placing the framework in a hydrogel and then freeze-drying the hydrogel so that the solid xerogel is coated on the framework; or spraying the hydrogel onto the waist 1 and then obtaining the solid xerogel coated on the framework by solvent evaporation. The hydrogel is selected from a liquid degradable material, preferably a block or cross-linked copolymer consisting of at least one aliphatic polymer and at least one water-soluble polymer or of at least one amphiphilic polymer and at least one aliphatic or water-soluble polymer. For example, it may be a PLA block copolymer (e.g., PLA Pluronic® F127) or a PEG-PLGA-PEG, PLGA-PEG-PLGA, PCLA-PEG-PCLA or Dex-PCL-HEMA copolymer or the like. The degradable material of the hydrogel is preferred to have a weight average molecular weight of 10⁴-10⁶ Dₐ.

The blood-blocking membranes include a proximal blood-blocking membrane 3 located at a proximal end of the framework and a distal blood-blocking membrane 4 located at a distal end of the framework. Additionally, they are secured at opposing ends of the waist 1 or at inner sides of the barbs 2, 5. In this embodiment, the blood-blocking membranes are sutured and secured at both ends of the waist 1 using degradable sutures so as to ensure tight closure by the blood-blocking membranes.

The step of coating the solid xerogel onto the waist 1 and the step of securing the blood-blocking membranes 3, 4 may be interchanged and, depending on where the solid xerogel is coating or where the blood-blocking membranes are secured, may be carried out either before or after the fabrication of the framework waist 1 or of the barbs 2, 5, and the present invention is not so limited. Besides the waist 1, the solid xerogel may be also coated on the barbs 2, 5 or on the blood-blocking membranes 3, 4.

After the fabrication of the occlusion device 8 is completed, it is delivered through a catheter to close the cardiac defect, as specifically shown in Figs. 4 and 5. At first, after the occlusion device 8 is crimped and load into the delivery catheter 10, it is advanced to the site of the cardiac defect. When reaching the target site, the distal barbs 5 are first pushed out of the delivery catheter 10 so that they expand and engage the tissue wall 9 around the defect, as shown in Fig. 5. Subsequently, the delivery catheter 10 is retracted, resulting in the release of the waist 1 and the proximal barbs 2. As a result, the proximal barbs 2 also expand and engage the tissue wall 9. Since the waist 1 of the occlusion device 8 in the released configuration has an inner diameter that is much greater than an inner diameter of the defect 11, the waist 1 is compressed by the tissue wall 9 and closely fitted against it. At this point, the deployment of the occlusion device 8 is completed. In the course of the implantation of the occlusion device 8, upon coming into contact with the blood, the xerogel on the framework will absorb water therein and initially swell into the hydrogel. After the completion of the deployment of the occlusion device 8, the hydrogel will additionally absorb water and gradually fill up and finally close the defect 11.

In summary, compared to the prior art, the occlusion device of the present invention offers the following benefits and advantages:
1. In lieu of the conventional bulky double-disc design, the framework of the occlusion device according to the present application is constructed from a waist and much less bulky barbs connected thereto, which make the occlusion device compact in size while still allowing it to be anchored to a target defect. The barbs are stable in structure without proneness to reversion or deformation and are thus able to firmly fix the framework in place without any potential displacement. The solid xerogel coated on the waist will swell when it comes into contact with blood and absorbs water therein. As a result, it will finally come into contact with, and adhesively bond to, the tissue wall around the defect, providing an even stronger protection to occlusion device from displacement after it is deployed. Further, due to greatly reduced footprints of the components for anchoring the occlusion device from both sides of the defect, the occlusion device of the present invention can be more extensively used in patients with defects having relatively short distance from the coronary sinus, superior vena cava, inferior vena cava, pulmonary vein, atrioventricular valves and the like, resulting in an expansion in the scope of application of minimally-invasive interventional treatment to cardiac septal defects.
2. Compared to the conventional bulky double-disc design, it has a significantly reduced outer diameter and overall size and thus can be delivered within a thinner delivery catheter with a smaller diameter. This can reduce damage to the wall of a blood vessel in which the catheter is inserted to deliver the occlusion device and hence reduce inflammation in the blood vessel. Further, such thinner delivery catheters can be used in younger cardiac septal defect patients, resulting in an additional expansion in the scope of application of minimally-invasive interventional treatment to cardiac septal defects.
3. Compared to the conventional bulky double-disc design, the occlusion device according to the present application saves about 50% in material used for framework, leading to significant cost reductions.
4. In preferred embodiments of the present invention, the occlusion device is made of more flexible degradable materials, including polycaprolactone (PCL), polygly-colide (PGA), polylactic acid (PLA), polydioxanone (PDO), polyamino acid (PAA), the aforementioned copolymer and/or blend, a degradable zinc alloy or a degradable magnesium alloy, from which the framework is made, and polycaprolactone (PCL), polyglycolide (PGA), polylactic acid (PLA), polydioxanone (PDO), polyamino acid (PAA) or the aforementioned copolymer and/or blend, from which the blood-blocking membranes are made. As a result, good deformability is achieved, which allows the solid xerogel that have contacted the blood and swollen to fill up the defect even when the defect has any complicated irregular shape, for example, with asymmetric opposing sides. In this way, effective closure can always be obtained, and the scope of application of minimally-invasive interventional treatment can be further extended to patients with complicated defects.
5. In preferred embodiments of the present invention, the entire occlusion device, including the framework, blood-blocking membranes and solid xerogel, may be made of degradable materials. As a result, the occlusion device will gradually disappear after endothelialization of the defect and is thus much safer to the human body. Additionally, since the occlusion device is smaller in size and uses a reduced amount of material, its degradation produces much less products that may cause complications.
6. In contrast to conventional degradable occlusion device, most of which have to incorporate sophisticated locking structures that require complex fabrication processes, the occlusion device of the present invention is simple in structure and can be easily fabricated while providing excellent performance.

While the present invention has been described above in terms of preferred embodiments, it is not limited to the embodiments disclosed herein. Any person of skill in the art can make changes and modifications without departing from the scope or spirit of the invention. Accordingly, the true scope of the invention is in-tended to be as defined by the appended claims.

## Claims

1. An occlusion device, comprising a framework and blood-blocking membranes, the framework comprising a waist and barbs connected to at least one end of the waist, the waist being coated with a solid xerogel, the blood-blocking membranes being secured at least one end of the waist and/or at inner sides of the barbs.

2. The occlusion device of claim 1, wherein the solid xerogel is made of a degradable material.

3. The occlusion device of claim 2, wherein the degradable material of the solid xerogel is selected from block or cross-linked copolymers, each consisting of at least one aliphatic polymer and at least one water-soluble polymer or of at least one amphiphilic polymer and at least one aliphatic or water-soluble polymer.

4. The occlusion device of claim 3, wherein the aliphatic polymer is selected from polylactic acid or a copolymer thereof, or from polycaprolactone or a copolymer thereof, and wherein the water-soluble polymer is selected from polyacrylic acid or polyethylene glycol, and wherein the amphiphilic polymer is selected from poly (N-isopropylacrylamide) or a copolymer thereof.

5. The occlusion device of claim 3, wherein the degradable material of the solid xerogel has a weight average molecular weight in a range of 10⁴-10⁶ Da.

6. The occlusion device of any of claims 1 to 5, wherein the framework and/or the blood-blocking membranes are/is each made of a degradable material.

7. The occlusion device of claim 6, wherein the degradable material of the framework is selected from a degradable magnesium alloy, a degradable zinc alloy or one or a copolymer and/or blend of more of polylactic acid, polyglycolide, polycaprolactone, polydioxanone and polyamino acid, and wherein the degradable material of the blood-blocking membranes is selected from one or a copolymer and/or blend of more of polylactic acid, polyglycolide, polycaprolactone, polydioxanone and polyamino acid.

8. The occlusion device of any of claims 1 to 5, wherein the waist is a meshed tubular structure or a hollow tubular structure.

9. The occlusion device of any of claims 1 to 5, wherein the waist has an axial length in a range of 3.5-5.5 mm and an inner diameter in a range of 2-60 mm.

10. The occlusion device of any of claims 1 to 5, wherein each of the barbs extends along a radial direction of the framework from a junction with the waist away from the waist and comprises at least one inwardly bent section pointing toward the waist.

11. The occlusion device of claim 10, wherein each of the barbs comprises a plurality of inwardly bent sections including at least one semicircular curved bent section and/or at least one hook-shaped bent section.

12. The occlusion device of any of claims 1 to 5, wherein the barbs are uniformly or non-uniformly arranged across a circumference of the waist or across one half or three quarters of the waist.

13. The occlusion device of any of claims 1 to 5, wherein the barbs are arranged on both ends of the waist, and wherein magnetic poles that attract each other are arranged on the barbs which are at symmetrical locations of proximal and distal ends of the waist respectively.

14. A preparation method for the occlusion device of any of claims 1 to 13, comprising steps of:
forming the waist of the framework by braiding filaments or laser-cutting a tube;
forming the barbs at at least one end of the waist by heat-setting;
securing the blood-blocking membranes at at least one end of the waist and/or at inner sides of the barbs; and
coating the solid xerogel onto the waist.

15. The preparation method of claim 14, wherein the waist is formed by braiding filaments, and wherein the barbs are formed by heat-setting portions of the filaments reserved at the end(s) of the waist.

16. The preparation method of claim 14, wherein the heat-setting comprises bending the filaments toward the waist by an over-pressure and thus imparting inward pre-stress to the barbs.

17. The preparation method of claim 14, wherein coating the solid xerogel onto the waist comprises: placing the waist in a hydrogel and forming the solid xerogel over the waist by freeze-drying the hydrogel; or spraying the hydrogel onto the waist and obtaining the solid xerogel by drying the hydrogel through solvent evaporation; or spraying the hydrogel onto the filaments or the tube from which the waist is formed and obtaining the solid xerogel by drying the hydrogel through solvent evaporation.

18. The preparation method of claim 14, wherein the blood-blocking membrane are secured at at least one end of the waist and/or at inner sides of the barbs by suturing, hot-melting or gluing.
